**Europäisches Patentamt**

**European Patent Office**

**Office européen des brevets**

(19)

(11) Veröffentlichungsnummer: **0 113 437**
**B1**

(12) # EUROPÄISCHE PATENTSCHRIFT

(45) Veröffentlichungstag der Patentschrift:
03.02.88

(51) Int. Cl.⁴: **G 03 G  5/06,** G 03 G  5/14,
C 07 C  109/12, C 07 D  231/06,
C 09 B  5/62

(21) Anmeldenummer: **83112181.9**

(22) Anmeldetag: **03.12.83**

(54) Elektrophotographisches Aufzeichnungsmaterial.

(30) Priorität: **09.12.82  DE 3246036**

(43) Veröffentlichungstag der Anmeldung:
**18.07.84 Patentblatt 84/29**

(45) Bekanntmachung des Hinweises auf die Patenterteilung:
**03.02.88 Patentblatt 88/5**

(84) Benannte Vertragsstaaten:
**DE FR GB NL**

(56) Entgegenhaltungen:
**DE-A-2 314 051**
**DE-A-2 919 791**
**DE-A-2 924 865**
**DE-A-3 019 326**
**US-A-3 871 882**
**US-A-4 030 923**

(73) Patentinhaber: **HOECHST AKTIENGESELLSCHAFT,**
**Postfach 80 03 20, D-6230 Frankfurt am Main 80**
**(DE)**

(72) Erfinder: **Moroni, Wilko, Dr., Husarenstrasse 27,**
**D-6900 Heidelberg (DE)**

EP 0 113 437 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die vorliegende Erfindung betrifft ein elektrophotographisches Aufzeichnungsmaterial aus einem elektrisch leitenden Schichtträger und mindestens einer bindemittelhaltigen photoleitfähigen Schicht, die ein Perylentetracarbonsäurediimid als Ladungsträger erzeugende Verbindung und ein Hydrazon- bzw. Pyrazolinderivat als Ladungen transportierende Verbindung enthält.

Es ist bekannt (DE-A-2 919 791 = US-A-4 278 747), elektrophotographische Aufzeichnungsmaterialien einzusetzen, die in der organischen Photoleiterschicht die verschiedensten Hydrazonverbindungen mit einer aromatischen Hydrocarbylgruppe oder einer aromatischen heterocyclischen Gruppe enthalten. Die Photoleiterschicht kann zusätzlich Farbstoffe und/oder Elektronenakzeptoren, die mit der Hydrazonverbindung einen Ladungsübertragungskomplex bilden, enthalten. Sie kann auch mit Materialien wie Selen, Selenverbindungen, Cadmiumsulfid, Phthalocyaninpigment, Perinon- oder Perylenpigment, Bisazo- und Cyaninpigment kombiniert sein.

Es ist auch elektrophotographisches Aufzeichnungsmaterial bekannt (US-A-4 030 923 = DE-A-2 654 873) mit einer Photoleiterschicht, die Triarylpyrazolin in Verbindung mit bindemittelgemischen als Ladungstransportschicht enthält. Hochabriebfeste Materialien erhält man dann, wenn in Kombination mit Bisazopigmenten Polycarbonate als Bindemittel eingesetzt werden.

Weiterhin ist bekannt (DE-C-2 924 865 = US-A-4 278 746), in elektrophotographischen Aufzeichnungsmaterialien mit leitendem Schichtträger bindemittelhaltige Ladungstransportschichten mit Pyrazolinverbindungen zu verwenden, die mit den verschiedensten Ladungsträger erzeugenden Substanzen und polymeren Bindemitteln kombiniert werden können. Es ist auch bekannt (US-A-3 904 407 = DE-A-2 108 992), Perylenpigmente, insbesondere Perylentetracarbonsäurederivate, als Ladungsträger erzeugende Verbindungen in photoleitfähigen Systemen zu verwenden. Aus der DE-A-2 237 539 (= US-A-3 871 882) ist die Kombination von Perylentetracarbonsäurediimiden mit Hydrazon- und Pyrazolin-Derivaten als Ladungsträger erzeugende bzw. Ladungen transportierende Verbindungen bekannt. Perylenpigmente sind auch aus US-A-3 972 717 (= DE-A-2 314 051) bekannt. Aus DE-A-3 019 326 geht die Verwendung von N,N'-Bis-(3-methoxypropyl)-perylentetracarbonsäurediimid bestimmter Kristallmodifikation hervor.

Die bekannten Aufzeichnungsmaterialien zeigen, insbesondere in Photoleiterdoppelschichtanordung aus Ladungsträger erzeugender Schicht und Ladungstransportschicht, gute Lichtempfindlichkeiten. Ihre mechanischen Beständigkeiten werden zusätzlich durch Einsatz von verträglichen, hochpolymeren Bindemitteln gefördert. So können Aufdampfschichten aus Perylentetracarbonsäurederivaten mit Ladungstransportschichten kombiniert werden, die Polyester- oder Celluloseester-Bindemittel enthalten. Bei guter Lichtempfindlichkeit weisen solche Aufzeichnungsmaterialien jedoch eine gewisse Sprödigkeit auf, oder sie führen zu feinen Haarrißbildungen der Schichten oder sie sind nicht genügend abriebfest. Werden jedoch in entsprechenden Aufzeichnungsmaterialien schlagzähe, hochabriebfeste Polycarbonate oder Polyepoxide eingesetzt, dann verliert man entweder an guter Photoleitfähigkeit oder man beobachtet Unverträglichkeiten, die das Aufzeichnungsmaterial für einen praktischen Gebrauch in cyclischen Kopierprozessen unbrauchbar machen.

Durch den bekannten Stand der Technik ist es insgesamt zwar möglich, gut lichtempfindliche elektrophotographische Aufzeichnungsmaterialien mit längerwelliger Empfindlichkeit zu erhalten, deren längerwellige Empfindlichkeit, wie z. B. bei Bisazopigmenten, man jedoch für Bürokopiergeräte wieder wegfiltern muß, um eine praxisgerechte Farbwiedergabe der Originale zu erzielen. Als weiterer Nachteil gilt, daß Photoleiterschichten dieser Art nur dann in Doppelschichtanordnung herstellbar sind, wenn die Schicht mit der Ladungsträger erzeugenden Verbindung in Dispersion mit einem Bindemittel vorliegt, da man Bisazopigmente nicht ohne Zersetzung bei höherer Temperatur auftragen kann.

Es war deshalb Aufgabe der vorliegenden Erfindung, ein hochlichtempfindliches elektrophotographisches Aufzeichnungsmaterial zu schaffen, das in höchstem Maße abriebfest ist und gut auf dem leitenden Schichtträger haftet.

Die Lösung dieser Aufgabe geht aus von einem elektrophotographischen Aufzeichnungsmaterial aus einem elektrisch leitenden Schichtträger und mindestens einer bindemittelhaltigen photoleitfähigen Schicht, die ein Perylentetracarbonsäurediimid als Ladungsträger erzeugende Verbindung und ein Hydrazon- bzw. Pyrazolinderivat als Ladungen transportierende Verbindung enthält. Das Material ist dadurch gekennzeichnet, daß die Ladungen transportierende Verbindung 1,5-Diphenyl-3-p-methoxyphenylpyrazolin, 1-Phenyl-3-p-methylstyryl-5-p-tolyl-pyrazolin, ein Kondensationsprodukt aus 1,1-Diphenyl-hydrazin und Anisaldehyd oder ein Kondensationsprodukt aus 1,1-Diphenylhydrazin und p-Tolylaldehyd ist.

Als Ladungsträger erzeugende Verbindungen kommen erfindungsgemäß N,N'-Dimethyl-perylen-3,4,9,10-tetracarbonsäurediimid (Formel A), N,N'-Bis-(3-methoxypropyl)-perylen-3,4,9,10-tetracarbonsäurediimid (Formel B) und das Kondensationsprodukt aus Perylentetracarbonsäureanhydrid und o-Phenylendiamin (Formel C) in Betracht.

Die Verbindungen sind bekannt und können nach den Angaben in DE-A-2 237 539, DE-A-3 019 326 und US-A-3 972 717 hergestellt und verwendet werden. Vorzugsweise werden sie durch Aufdampfen im Vakuum auf den Schichtträger aufgebracht.

Erfindungsgemäß werden als Ladungen transportierende Verbindungen 1,5-Diphenyl-3-p-methoxyphenyl-pyrazolin (Formel 1), 1-Phenyl-3-p-methylstyryl-5-p-tolyl-pyrazolin (Formel 2), ein Kondensationsprodukt aus 1,1-Diphenyl-hydrazin und Anisaldehyd (Formel 3) und/oder ein Kondensationsprodukt aus 1,1-Diphenylhydrazin und p-Tolylaldehyd (Formel 4) eingesetzt.

Die Herstellung der Pyrazolinverbindungen erfolgt analog den Angaben in DE-B-1 060 714, Seite 1 (=US-A-3 180 729), die Kondensation der Hydrazine ist in DE-A-2 919 791, Seite 8 (=US-A-4 278 747) beschrieben.

Durch die Erfindung wird erreicht, daß man eine optimale Übereinstimmung der elektronischen Potentiale und Konfigurationen von Ladungen transportierenden Verbindungen mit den spektral besonders günstigen Perylentetracarbonsäurediimiden als Ladungsträger erzeugende Verbindungen erzielt, wenn man das -Elektronensystem der erfindungsgemäßen Hydrazon- und Pyrazolinverbindungen durch entsprechend Elektronen ziehende oder donierende Substituenten beeinflußt. Es hat sich erfindungsgemäß gezeigt, daß nur ganz bestimmt substituierte Ladungen transportierende Verbindungen mit den Perylentetracarbonsäurediimiden in Verbindung mit einem Bindemittel hochlichtempfindliche Photoleiterschichten für elektrophotographisches Aufzeichnungsmaterial ergeben, wobei die Vorteile der Perylentetracarbonsäurediimide hinsichtlich ihrer Herstellung, spektralen Empfindlichkeit, Aufdampfbarkeit und Dispergierfähigkeit aus den genannten Druckschriften bekannt sind.

Die Oberflächen der erfindungsgemäßen Aufzeichnungsmaterialien können hochabriebfest und gut haftend erstellt werden und erreichen Empfindlichkeiten $E_{1/2}$ von unter 2,5 $\mu J/cm^2$.

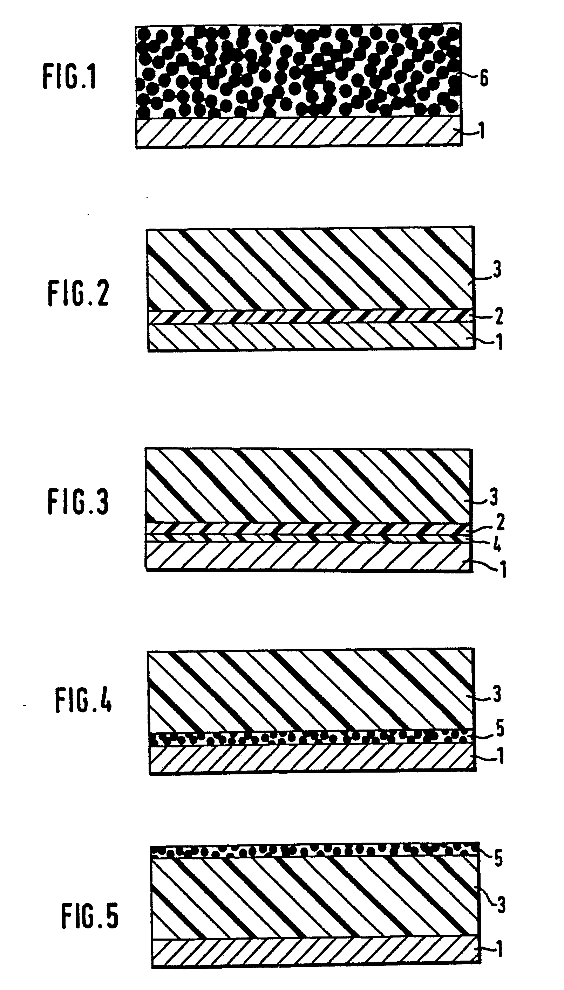

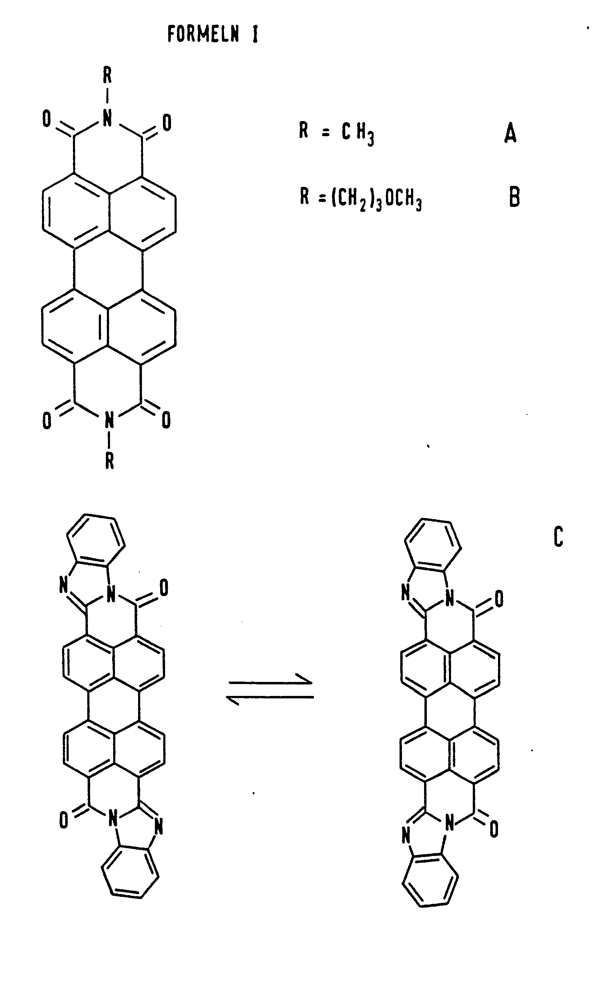

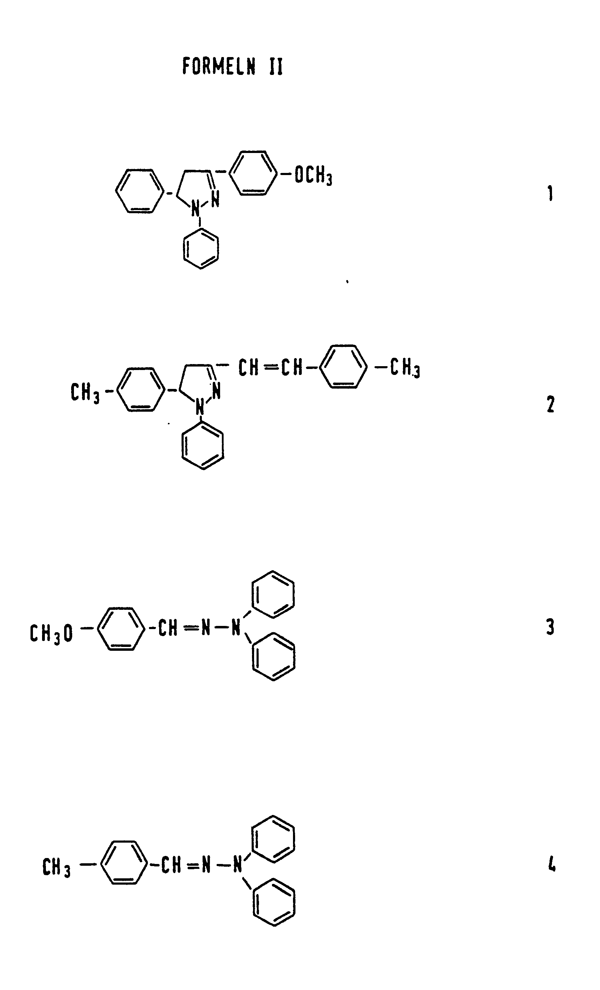

Der in der Elektrophotographie allgemein bekannte Aufbau des erfindungsgemäßen elektrophotographischen Aufzeichnungsmaterials wird anhand der beigefügten Figuren 1 bis 5 schematisch näher erläutert.

Mit Position 1 ist jeweils der elektrisch leitende Schichtträger angezeigt, Position 2 weist auf die Ladungsträger erzeugende Schicht hin, und mit Position 3 wird die Ladungstransportschicht angegeben. Position 4 gibt eine isolierende Zwischenschicht an, und mit Position 5 ist eine Schicht wiedergegeben, die eine Ladungsträger erzeugende Schicht in Dispersion mit einem Bindemittel darstellt. Unter Position 6 ist eine photoleitfähige Schicht in Dispersion aus Ladungen transportierender, Ladungen erzeugender Verbindung und Bindemittel usw. aufgezeichnet.

Die Einführung einer isolierenden Zwischenschicht, gegebenenfalls auch einer thermisch, anodisch oder chemisch erzeugten Aluminiumoxid-Zwischenschicht (Figur 3, Position 4), hat zum Ziel, die Ladungsträgerinjektion vom Metall in die Photoleiterschicht im Dunkeln herabzusetzen. Andererseits soll sie beim Belichtungsvorgang den Ladungsfluß nicht hindern. Die Zwischenschicht wirkt als Sperrschicht. Die Zwischenschicht dient gegebenenfalls auch dazu, die Haftung zwischen der Schichtträgeroberfläche und der Farbstoffschicht bzw. Photoleiterschicht zu verbessern.

Für die Zwischenschicht können unterschiedliche Natur- bzw. Kunstharzbindemittel verwendet werden, bevorzugt werden jedoch solche Materialien eingesetzt, die gut auf einer Metall-, speziell Aluminiumoberfläche, haften und beim nachfolgenden Anbringen weiterer Schichten wenig angelöst werden. Hierzu gehören Polyamidharze, Polyvinylphosphonsäure, Polyurethane, Polyesterharze oder spezifisch alkalilösliche Bindemittel, wie zum Beispiel Styrol-Maleinsäureanhydrid-Copolymerisate.

Die Dicke organischer Zwischenschichten kann bis zu 5 µm betragen, die einer Aluminiumoxid-Zwischenschicht liegt im allgemeinen im Bereich von 10 nm bis 5 µm.

Grundsätzlich zeigt für ein definiertes Pigment/Photoleiter/Bindemittel-System die Monodispersionsschichtanordnung nach Figur 1 die geringste Lichtempfindlichkeit, da hier die Trapwirkung für die erzeugten Ladungsträger am größten ist. Durch die Schichtanordnung wird aber auch die Polarität der Aufladung bestimmt. Während monodisperse Schichten nach Figur 1 sowohl positiv als auch negativ aufladbar sind, können Schichten in Doppelschichtanordnung mit p-Photoleitern (Fig. 2, 3, 4) nur negativ, und in inverser Anordnung (Fig. 5) nur positiv aufgeladen werden.

Die Monodispersionsschichtanordnung nach Figur 1 ist vorteilhaft für die Herstellung von Druckformen auf elektrophotographischem Wege, während die Anordnung in Mehrfachschicht vorteilhafte Eigenschaften für cyclisch arbeitende Verfahren, z. B. in Kopiergeräten, bringt.

Als elektrisch leitender Schichtträger kommt bevorzugt Aluminiumfolie, gegebenenfalls transparente, mit Aluminium bedampfte bzw. Aluminium-kaschierte Polyesterfolie zum Einsatz, jedoch kann jeder andere genügend leitend gemachte Schichtträger auch verwendet werden. Die Anordnung der photoleitfähigen Schicht kann auch auf einer Trommel, auf flexiblen Endlosbändern, zum Beispiel aus Nickel oder Stahl, oder auf Platten erfolgen.

Eine Schicht mit der Ladungen transportierenden Verbindung und Bindemittel weist ohne Zusatz der Perylentetracarbonsäurediimide im sichtbaren Bereich (420 - 750 nm) praktisch keine Lichtempfindlichkeit auf. Erst der Zusatz von Perylenpigment führt dazu, daß die Photonen des Lichts im Pigment angeregte Elektronenzustände und, unter dem Einfluß des elektrischen Feldes, Ladungsträger erzeugen, die von den Molekülen der Ladungstransportverbindung durch die Schicht transportiert werden.

Im Dunkeln besitzt das elektrophotographische Aufzeichnungsmaterial einen hohen elektrischen Widerstand von mehr als $10^{12} \Omega$. Es verhindert im Dunkeln das Abfließen der elektrostatischen Ladung. Das Abfließen erfolgt erst durch Lichteinwirkung.

Neben den erfindungsgemäßen, Ladungsträger erzeugenden Verbindungen sowie den Ladungen transportierenden Verbindungen beeinflußt das zugesetzte Bindemittel sowohl das mechanische Verhalten wie Abrieb, Flexibilität, Filmbildung etc. als auch in gewissem Umfang das elektrophotographische Verhalten wie Lichtempfindlichkeit, Restladung sowie cyclisches Verhalten.

Als Bindemittel werden filmbildende Verbindungen wie Polyesterharze, Polyvinylchlorid/Polyvinylacetat-Mischpolymerisate, Styrol-Maleinsäureanhydrid-Copolymerisate, Polycarbonate, Silikonharze, Polyurethane, Sulfonylurethane, Epoxidharze, Acrylate, Polyvinylacetate, Polystyrole eingesetzt. Außerdem werden thermisch nachvernetzende Bindemittelsysteme wie Reaktivharze, die sich aus einem äquivalenten Gemisch von hydroxylgruppenhaltigen Polyestern bzw. Polyethern und polyfunktionellen Isocyanaten zusammensetzen, polyisocyanatvernetzbare Acrylatharze, Melaminharze, ungesättigte Polyesterharze etc. erfolgreich angewandt.

Wegen der guten Lichtempfindlichkeit, der großen Flexibilität und insbesondere der guten Abriebfestigkeit ist der Einsatz von Polycarbonaten besonders bevorzugt.

Das Mischungsverhältnis der ladungstransportierenden Verbindung zu dem Bindemittel kann variieren. Jedoch sind durch die Forderung nach maximaler Lichtempfindlichkeit, d. h. möglichst großem Anteil an Ladungen transportierender Verbindung, und nach zu vermeidender Auskristallisation sowie Erhöhung der Flexibilität, d. h. möglichst großem Anteil an Bindemitteln, relativ bestimmte Grenzen gesetzt. Es hat sich allgemein ein Mischungsverhältnis von etwa 1 : 1 Gewichtsteilen als bevorzugt erwiesen, jedoch sind auch Verhältnisse zwischen 4 : 1 bis 1 : 2 geeignet.

Die jeweiligen Erfordernisse eines Kopiergerätes an die elektrophotographischen sowie mechanischen Eigenschaften des Aufzeichnungsmaterials können durch unterschiedliche Einstellung der Schichten, zum Beispiel durch die Viskosität der Bindemittel, Anteil der Ladungen transportierenden Verbindung, in einem weiten Rahmen erfüllt werden.

Für die optimale Lichtempfindlichkeit ist auch die Schichtdicke eine wichtige Größe: Schichtdicken zwischen etwa 3 und 20 µm werden im allgemeinen eingesetzt. Als besonders vorteilhaft hat sich ein Dickenbereich von 4 bis 12 µm erwiesen. Doch können, wenn es die mechanischen Erfordernisse sowie die elektrophotographischen Parameter (Aufladungs- und Entwicklungsstation) eines Kopiergerätes zulassen, die angegebenen Grenzen nach oben oder unten fallweise erweitert werden.

Für die Beurteilung der Ladungen transportierenden Verbindungen und der Ladungsträger erzeugenden Verbindungen ist es unerheblich, in welcher Schichtanordnung sie getestet werden. Die Verwendung kann in allen Schichtanordnungen gemäß Figur 1 bis Figur 5 erfolgen. Die Erfindung wird anhand der Beispiele in Doppelschichtanordnung gemäß Figur 2 erläutert, ohne sie hierauf zu beschränken.

Wie die Beispiele zeigen, sind die erfindungsgemäßen Hydrazon- und Pyrazolin-Verbindungen der Formeln 1 bis 4 ausgezeichnete Ladungen transportierende Verbindungen.

Die Messung der Lichtempfindlichkeit wird wie folgt durchgeführt:

Zur Ermittlung der Hellentladungskurven bewegt sich die Meßprobe auf einem sich drehenden Teller durch eine Aufladevorrichtung hindurch zur Belichtungsstation, wo sie mit einer Xenonlampe XBO 150 der Firma Osram kontinuierlich belichtet wird. Ein Wärmeabsorptionsglas und ein Neutralfilter mit 12 % Transparenz sind der Lampe vorgeschaltet. Die Lichtintensität in der Meßebene liegt im Bereich von 50 bis 100 µW/cm²; sie wird unmittelbar nach Ermittlung der Hellabfallkurve mit einem Optometer gemessen. Die Aufladungshöhe und die photoinduzierte Hellabfallkurve werden über ein Elektrometer durch eine transparente Sonde oszillographisch aufgezeichnet. Die photoleitfähige Schicht wird durch die Aufladungshöhe ($U_0$) und diejenige Zeit ($T_{1/2}$) charakterisiert, nach der die Hälfte der Aufladung ($U_{0/2}$) erreicht ist. Das Produkt aus $T_{1/2}$ und der gemessenen Lichtintensität $I[\mu W/cm^2]$ ist die Halbwertsenergie $E_{1/2}[\mu J/cm^2]$. $U_R$ gibt die Restladung an, die nach 0,1 s Belichtung noch vorhanden ist.

**Beispiel 1:**

Auf eine Aluminium-bedampfte Polyesterfolie wird N,N'-Dimethylperylen-3,4,9,10-tetracarbonsäurediimid (Formel A), dessen spektrale Lichtabsorption von 430 bis etwa 600 nm reicht, in einer Vakuum-Bedampfungsanlage bei $1,33 \times 10^{-7}$ bis $10^{-8}$ bar und ca. 280°C aufgedampft. Die homogen aufgedampfte Schicht besitzt ein Schichtgewicht von ca. 150 mg/m² und deckt vollständig den Schichtträger ab.

Auf die Aufdampfschicht wird eine Lösung aus gleichen Gewichtsteilen des Hydrazons der Formel 3 und eines Polycarbonatharzes (Makrolon® 2045, Bayer) in Tetrahydrofuran (THF) geschleudert. Die Schicht wird etwa 5 Minuten lang bei ca. 110°C in einem Umlufttrockenschrank getrocknet. Die Schicht ist glatt und gut haftend. Die Lichtempfindlichkeit wird gemäß der oben beschriebenen Charakterisierungsmethode bestimmt zu:

| $(-) U_o(V)$ | $(-) U_R(V)$ | $E_{1/2}$ |
|---|---|---|
| 733 | 186 | 1,15 |
| 560 | 140 | 1,35 |

Verwendet man unter sonst gleichen Bedingungen statt des angegebenen Hydrazons die Verbindungen
2-Vinyl-4 (2'-chlorphenyl)-5 (4'-diethylaminophenyl)-oxazol-1,3 oder
2-Phenyl-4 (2'-chlorphenyl)-5 (4'-diethylaminophenyl)-oxazol-1,3,
analog DE-PS 11 20 875 = US-PS 3 257 203, oder
2,5-Bis-(4'-diethylaminophenyl)-oxdiazol-1,3,4 gemäß DE-PS 10 58 836 = US-PS 3 189 447,
dann erhält man folgende Werte:

| $(-) U_o(V)$ | $(-) U_R(V)$ | $E_{1/2}$ |
|---|---|---|
| 747 | | > 30 |
| 560 | 180 | 2,56 |
| 620 | 173 | 1,76 |

**Beispiele 2 bis 4:**

Auf die aufgedampften, dunkelroten Schichten der Verbindung nach Formel A, hergestellt wie in Beispiel 1, werden Lösungen aus gleichen Gewichtsteilen der Hydrazon- und Pyrazolin-Verbindungen mit den Formeln 1, 2 und 4 und dem Bindemittel nach Beispiel 1 in THF aufgeschleudert, getrocknet und die Lichtempfindlichkeit der ca. 9 bis 10 μm dicken Schichten gemessen.

| Verbindung | $(-)U_o(V)$ | $(-)U_R(V)$ | $E_{1/2}$ |
|---|---|---|---|
| 1 | 747 | 137 | 2,37 |
| 2 | 474 | 93 | 2,51 |
| 4 | 727 | 180 | 1,73 |

**Beispiele 5 bis 9:**

Auf eine Aluminium-bedampfte Polyesterfolie wird N,N'-Bis(3-methoxypropyl)perylen-3,4,9,10-tetracarbonsäurediimid (Formel B) bei $10^{-7}$ bis $10^{-8}$ bar innerhalb von 2 Minuten bei 180 bis 220°C aufgedampft. Unter diesen Bedingungen bildet sich die blaugrüne, stabile Modifikation der Verbindung, die das Licht zwischen 430 und 650 nm absorbiert.

Auf diese homogen aufgedampfte Schicht mit einem Schichtgewicht von ca. 200 g/m² werden Lösungen aus gleichen Gewichtsteilen der Verbindungen mit den Formeln 1 bis 4 und Polycarbonat in THF aufgeschleudert und getrocknet. Die ca. 9 bis 10 μm dicken Schichten ergeben folgende Lichtempfindlichkeiten:

| Verbindung | $(-)U_o(V)$ | $(-)U_R(V)$ | $E_{1/2}$ |
|---|---|---|---|
| 1 | 713 | 186 | 1,62 |
| 2 | 687 | 153 | 1,71 |
| 3 | 740 | 180 | 1,46 |
| 4 | 800 | 226 | 1,37 |

Verwendet man vergleichsweise unter sonst gleichen Versuchsbedingungen die Verbindungen
2-(4'-Diethylaminophenyl-4-chlor-5-(4'-methoxyphenyl)-oxazol-1,3,
anaolg DE-C-1 120 875 = US-A-3 257 203, so erhält man folgende Werte:

| $(-)U_o$ | $(-)U_R(V)$ | $E_{1/2}$ |
|---|---|---|
| 620 | 206 | 2,04 |

Verwendet man vergleichsweise statt der beschriebenen Ladungstransportschichten eine Schicht bestehend aus gleichen Gewichtsteilen eines Polyesters als Bindemittel (Dynapol® 206, Dynamit Nobel) und des in Beispiel 1 genannten Vinyloxazols bei sonst gleichen Versuchsbedingungen, dann erhält man folgende werte:

| $(-)U_o(V)$ | $(-)U_R(V)$ | $E_{1/2}$ |
|---|---|---|
| 620 | 200 | 3,04 |

**Beispiele 10 bis 11:**

Auf eine Aluminium-bedampfte Polyesterfolie wird die Verbindung nach Formel C, die praktisch über den gesamten sichtbaren Bereich das Licht absorbiert (420 bis 750 nm), in einer Vakuum-Bedampfungsanlage bei $8 \times 10^{-5}$ bis $10^{-4}$ mbar und einer Heiztemperatur von 350 bis 370°C innerhalb von 2 bis 3 Minuten aufgedampft. Das Schichtgewicht beträgt dann etwa 200 mg/m².

Auf diese violetten Aufdampfungsschichen werden mit den Verbindungen der Formeln 3 und 4 und Polycarbonat als Bindemittel entsprechend den oben beschriebenen Beispielen ca. 9 bis 10 μm dicke Schichten aufgebracht. Die Messung der Lichtempfindlichkeit ergibt folgende Werte:

| Verbindung | $(-)U_o(V)$ | $(-)U_R(V)$ | $E_{1/2}$ |
|---|---|---|---|
| 3 | 426 | 140 | 0,95 |
| 4 | 440 | 146 | 1,62 |

## Patentansprüche

1. Elektrophotographisches Aufzeichnungsmaterial aus einem elektrisch leitenden Schichtträger und mindestens einer bindemittelhaltigen photoleitfähigen Schicht, die ein Perylentetracarbonsäurediimid als Ladungsträger erzeugende Verbindung und ein Hydrazon- bzw. Pyrazolinderivat als Ladungen transportierende Verbindung enthält, dadurch gekennzeichnet, daß die Ladungen transportierende Verbindung 1,5-Diphenyl-3-p-methoxyphenyl-pyrazolin, 1-Phenyl-3-p-methyl-styryl-5-p-tolyl-pyrazolin, ein Kondensationsprodukt aus 1,1-Diphenyl-hydrazin und Anisaldehyd oder ein Kondensationsprodukt aus 1,1-Diphenylhydrazin und p-Tolylaldehyd ist.

2. Aufzeichnungsmaterial nach Anspruch 1, dadurch gekennzeichnet, daß die Ladungsträger erzeugende Verbindung N,N'-Dimethyl-perylen-3,4,9,10-tetracarbonsäurediimid ist.

3. Aufzeichnungsmaterial nach Anspruch 1, dadurch gekennzeichnet, daß die Ladungsträger erzeugende Verbindung N,N-Bis-(3-methoxypropyl)-perylen-3,4,9,10-tetracarbonsäurediimid ist.

4. Aufzeichnungsmaterial nach Anspruch 1, dadurch gekennzeichnet, daß die Ladungsträger erzeugende Verbindung das Kondensationsprodukt aus Perylentetracarbonsäureanhydrid und o-Phenylendiamin ist.

5. Aufzeichnungsmaterial nach Anspruch 1, dadurch gekennzeichnet, daß das Bindemittel der photoleitfähigen Schicht ein Polycarbonat ist.

6. Aufzeichnungsmaterial nach Anspruch 1, dadurch gekennzeichnet, daß die photoleitfähige Schicht aus Ladungsträger erzeugender Schicht und Ladungstransportschicht besteht.

7. Aufzeichnungsmaterial nach Anspruch 1, dadurch gekennzeichnet, daß eine isolierende Zwischenschicht vorhanden ist.

8. Aufzeichnungsmaterial nach den Ansprüchen 1 bis 4, dadurch gekennzeichnet, daß das Perylentetracarbonsäurediimid durch Aufdampfen im Vakuum aufgebracht ist.

## Claims

1. An electrophotographic recording material made of an electrically conductive support and at least one binder-containing photoconductive layer which contains a perylenetetracarboxylic acid derivative as a compound which produces charge carriers and a hydrazone or pyrazoline derivative as a compound which transports charges, wherein the charge-transporting compound is 1,5-diphenyl-3-p-methoxyphenylpyrazoline, 1-phenyl-3-p-methylstyryl-5-p-tolyl-pyrazoline, a condensation product of 1,1-diphenylhydrazine and anisaldehyde or a condensation product of 1,1-diphenylhydrazine and p-tolylaldehyde.

2. A recording material as claimed in claim 1, wherein the compound which produces charge carriers is N,N'-dimethylperylene-3,4,9,10-tetracarboxylic acid diimide.

3. A recording material as claimed in claim 1, wherein the compound which produces charge carriers is N,N'-bis -(3-methoxypropyl)-perylene-3,4,9,10-tetracarboxylic acid diimide.

4. A recording material as claimed in claim 1, wherein the compound which produces charge carriers is the condensation product of perylenetetracarboxylic acid anhydride and o-phenylene-diamine.

5. A recording material as claimed in claim 1, wherein the binder of the photoconductive layer is a polycarbonate.

6. A recording material as claimed in claim 1, wherein the photoconductive layer comprises a layer which produces charge carriers and a charge transport layer.

7. A recording material as claimed in claim 1, wherein an insulating interlayer is present.

8. A recording material as claimed in claim 1 to 4, wherein the perylenetetracarboxylic acid diimide is applied by vacuum-sputtering.

## Revendications

1. Matériau de reproduction électrophotographique composé d'un support de couches conducteur de l'électricité, et d'au moins une couche photoconductrice, contenant un liant, qui contient un diimide d'acide pérylènetétracarboxylique en tant que composé producteur de porteurs de charge, et un dérivé hydrazone ou pyrazoline en tant que composé transporteur de charges, caractérisé en ce que le composé transportent de charges est la 1,5-diphényl-3-p-méthoxy-phénylpyrazoline, la 1-phényl-3-p-méthylstyryl-5-p-tolyl-pyrazoline, un produit de condensation de 1,1-diphénylhydrazine et anisaldéhyde, ou un produit de condensation de 1,1-diphénylhydrazine et p-tolylaldéhyde.

2. Matériau de reproduction selon la revendication 1, caractérisé en ce que le composé producteur de porteurs de charge est le diimide d'acide N,N'-diméthyl-pérylène-3,4,9, 10-tétracarboxylique.

3. Matériau de reproduction selon la revendication 1, caractérisé en ce que le composé producteur de porteurs de charge est le diimide d'acide N,N'-diméthyl-pérylène-3,4,9,10-tétracarboxylique.

4. Matériau de reproduction selon la revendication 1, caractérisé en ce que le composé producteur de porteurs de charge est le produit de condensation d'anhydride d'acide pérylènetétracarboxylique et de o-phénylènediamine.

5. Matériau de reproduction selon la revendication 1, caractérisé en ce que le liant de la couche potoconductrice est un polycarbonate.

6. Matériau de reproduction selon la revendication 1, caractérisé en ce que la couche photoconductrice est composée d'une couche productrice de porteurs de charge et d'une couche de transfert de charges.

7. Matériau de reproduction selon la revendication 1, caractérisé en ce qu'une couche intermédiaire isolante est présente.

8. Matériau de reproduction selon les revendications 1 à 4, caractérisé en ce que le diimide d'acide pérylènetétracarboxylique est appliqué par vaporisation sous vide.

FIG.1

FIG.2

FIG.3

FIG.4

FIG.5

FORMELN I

R = CH$_3$       A

R = (CH$_2$)$_3$OCH$_3$    B

C

# FORMELN II

1

2

3

4